(19) Europäisches Patentamt
European Patent Office
Office européen des brevets

(11) EP 1 996 543 B1

(12) EUROPÄISCHE PATENTSCHRIFT

(45) Veröffentlichungstag und Bekanntmachung des Hinweises auf die Patenterteilung:
**20.07.2011 Patentblatt 2011/29**

(21) Anmeldenummer: **07726567.6**

(22) Anmeldetag: **01.03.2007**

(51) Int Cl.:
***C07C 249/02*** (2006.01) ***A61Q 17/04*** (2006.01)
***A61K 8/40*** (2006.01) ***A61K 8/41*** (2006.01)

(86) Internationale Anmeldenummer:
**PCT/EP2007/051931**

(87) Internationale Veröffentlichungsnummer:
**WO 2007/104650 (20.09.2007 Gazette 2007/38)**

# (54) VERFAHREN ZUR HERSTELLUNG VON BENZOPHENONIMINEN

METHOD OF PRODUCING BENZOPHENONEIMINES

PROCEDE DE FABRICATION DE BENZOPHENONE IMINES

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **10.03.2006 EP 06110980**

(43) Veröffentlichungstag der Anmeldung:
**03.12.2008 Patentblatt 2008/49**

(73) Patentinhaber: **BASF SE**
**67056 Ludwigshafen (DE)**

(72) Erfinder:
- **HAHN, Thilo**
  **67292 Kirchheimbolanden (DE)**
- **HAESE, Frank**
  **63128 Dietzenbach (DE)**
- **KÖHLER, Ulrich**
  **68259 Mannheim (DE)**
- **SCHWAB, Ekkehard**
  **67434 Neustadt (DE)**
- **MELDER, Johann-Peter**
  **67459 Böhl-Iggelheim (DE)**
- **EBERHARDT, Jan**
  **68167 Mannheim (DE)**

(56) Entgegenhaltungen:
**EP-A1- 0 688 764 EP-A1- 0 713 861**

Bemerkungen:
Die Akte enthält technische Angaben, die nach dem Eingang der Anmeldung eingereicht wurden und die nicht in dieser Patentschrift enthalten sind.



Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft ein Verfahren zur Herstellung von Benzophenonimin (BPI) der allgemeinen Formel I

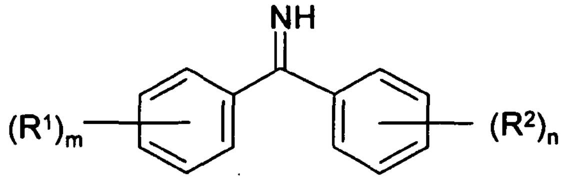

in der $R^1$ und $R^2$ $C_1$- bis $C_4$-Alkoxy, $C_1$- bis $C_2$-Alkylamin und $C_2$- bis $C_4$-Dialkylamin und m und n ganze Zahlen von 0 bis 5 bedeuten und $R^1$ und $R^2$ unabhängig voneinander gleich oder verschieden sein können, in dem man Benzophenon (BP) der allgemeinen Formel II

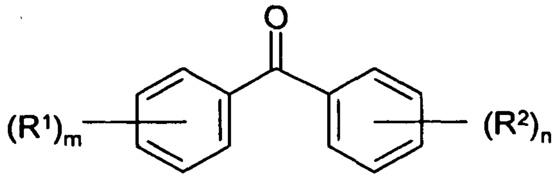

in der $R^1$, $R^2$, m und n die oben genannten Bedeutungen haben, in Ammoniak und in Gegenwart von Titandioxid umsetzt, wobei das Titandioxid im wesentlichen in der Anatas-Modifikation vorliegt.

Weitere Ausführungsformen der Erfindung sind den Ansprüchen, der Beschreibung und den Beispielen zu entnehmen. Es versteht sich, dass die vorstehend genannten und nachstehend noch zu erläuternden Merkmale des erfindungsgemäßen Gegenstands nicht nur in der jeweils angegebenen Kombination sondern auch in anderen Kombinationen verwendbar sind, ohne den Rahmen der Erfindung zu verlassen.

In der EP-A1-0688764 wird ein Verfahren zur Herstellung von aliphatischen und cycloaliphatischen Oximen offenbart, in dem man aliphatische oder cycloaliphatische Imine mit Sauerstoff oder einem sauerstoffhaltigen Gas in Gegenwart eines Katalysators behandelt. In der EP-A1-0688764 wird auch die Herstellung der entsprechenden aliphatischen bzw. cycloaliphatischen Imine durch Umsetzung der entsprechenden Alkanone mit Ammoniak an Heterogenkatalysatoren beschrieben.

In EP-A-0 713 861 wird Benzophenon in flüssigem Ammoniak in Gegenwart von Oxiden einer Reihe von Elementen oder deren Gemische, unter anderem Titan, bei Temperaturen von 80 bis 140°C und Drücken von 150 bis 250 bar umgesetzt. In der Patentschrift wird beschrieben, dass die Katalysatoren in Form von Pulvern (Rührautoklav) oder in Form von Tabletten oder Strängen (Rohrreaktoren) eingesetzt werden können. Wie aus den Beispielen hervorgeht, konnten unter Einsatz von Titanoxiden Umsätze an Benzophenon in einem kontinuierlichem Rohrreaktor zwischen 91 und 98% und Selektivitäten von 99% erreicht werden, wobei allerdings der Benzophenon-Umsatz mit steigender Belastung bei identischen Reaktionsbedingungen abnimmt.

Mittels der vorliegenden Erfindung sollte ein verbessertes Verfahren zur Herstellung von Benzophenoniminen entwickelt werden. Das erfindungsgemäße Verfahren ermöglicht hohe Umsätze von Benzophenonen mit guten Selektivitäten. Bei einer kontinuierlichen Herstellung kann die Belastung des Katalysators mit Edukt (Benzophenon) gegenüber herkömmlichen Verfahren ohne eine Verringerung des Umsatzes an Benzophenonen und ohne eine Verschlechterung der Selektivität erhöht werden. Dies ermöglicht beispielsweise eine Kapazitätserweiterung in bestehenden Anlagen bzw. eine Reduzierung des Reaktionsvolumens bei Neuanlagen und eine somit verbundene Reduktion der Investitionssumme.

Demgemäss wurde ein Verfahren gefunden, zur Herstellung von Benzophenonimin (BPI) der allgemeinen Formel I

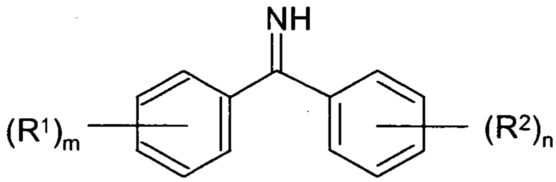

in der $R^1$ und $R^2$ $C_1$- bis $C_4$-Alkoxy, $C_1$- bis $C_2$-Alkylamin und $C_2$- bis $C_4$-Dialkylamin und m und n ganze Zahlen von 0 bis 5 bedeuten und $R^1$ und $R^2$ unabhängig voneinander gleich oder verschieden sein können, in dem man Benzophenon (BP) der allgemeinen Formel II

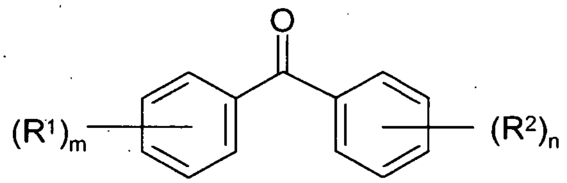

in der $R^1$, $R^2$, m und n die oben genannten Bedeutungen haben, in Ammoniak und in Gegenwart von Titandioxid umsetzt, wobei das Titandioxid im wesentlichen in der Anatas-Modifikation vorliegt.

Titandioxid kann in drei Modifikationen vorliegen: Rutil, Anatas sowie Brookit. Als Modifikation wird die Kristallstruktur bezeichnet, in der Titandioxid kristallisiert. Rutil und Anatas kristallisieren im tetragonalen Gitter während Brookit ein rhombisches Kristallsystem aufweist.

Die Kristallstruktur von Titandioxid kann analytisch mittels der Röntgendiffraktometrie bestimmt werden. Eine genaue Ausführungsvorschrift zur Bestimmung des Anatas-Anteils in Titandioxid ist in der Norm ASTM D 3720 aufgeführt.

Verfahren zur Herstellung von Titandioxid sind in der Literatur beschrieben [siehe hierzu Ullmann's Encyclopedia of Industrial Chemistry, 6th Edition, 2000 Electronic Release, Kapitel "Pigments, inorganic"; CEH Marketing Research Report, "Titanium Dioxide", 2005; Industrial Inorganic Pigments, edt. By G. Buxbaum u. G. Pfaff, Wiley VCH, Weinheim 2005]. Kommerzielle Prozesse zur Herstellung von Titandioxid sind der Sulfat-Prozess sowie der Chlorid-Prozess. Sowohl aus dem Sulfat-Prozess als auch aus dem Chlorid-Prozess kann Titandioxid sowohl in der Rutil- als auch in der Anatas-Modifikation erhalten werden, wobei Titandioxid, das im wesentlichen in der Anatas-Modifikation vorliegt, bevorzugt aus dem Sulfat-Prozess erhalten werden kann. Nach erfolgter Herstellung kann Titandioxid durch einen Nachbehandlungsschritt modifiziert werden. Geeignete Nachbehandlungsschritte können aus Abschnitt 2.1.3.4, "Industrial Inorganic Pigments", edt. by G. Buxbaum u. G. Pfaff, Wiley VCH, Weinheim 2005, entnommen werden.

Titandioxid ist in verschiedenen Modifikationen kommerziell erhältlich. Insbesondere eignen sich Titandioxide, die gemäß EN ISO 591-1:2000 dem Typ A (Anatas-Typ) und der Gruppe A1 zuzuordnen sind.

Das in dem erfindungsgemäßen Verfahren eingesetzte Titandioxid liegt im wesentlichen in der Anatas-Modifikation vor. Das eingesetzte Titandioxid kann aber auch ausschließlich in der Anatas-Modifikation vorliegen. Es ist jedoch auch möglich, dass das Titandioxid nicht vollständig in der Anatas-Modifikation vorliegt, sondern Anteile anderer Modifikationen, insbesondere Rutil, aufweist. Bevorzugter weise liegt das in dem erfindungsgemäßen Verfahren eingesetzte Titandioxid zu mindestens 50 Gew.% in der Anatas-Modifikation vor, vorteilhaft zu mindestens 80 Gew.% in der Anatas-Modifikation und besonders vorteilhaft zu mindestens 95 Gew.% in der Anatas-Modifikation vor, bezogen auf das insgesamt eingesetzte Titandioxid.

Das erfindungsgemäße Verfahren kann diskontinuierlich oder kontinuierlich betrieben werden.

Das Titandioxid kann als Pulver oder in Gestalt von Formkörpern, wie Strängen, Tabletten oder Granulat, eingesetzt werden.

Im diskontinuierlichen Verfahren wird Titandioxid bevorzugt als Pulver eingesetzt. Das Verhältnis von Titandioxid-Pulver zu Benzophenon der Formel II liegt im Allgemeinen in dem Bereich von 0,001 bis 5 kg Titandioxid/kg BP, bevorzugt in dem Bereich von 0,01 bis 3 kg Titandioxid/kg BP und besonders bevorzugt in dem Bereich von 0,5 bis 2 kg Titandioxid/kg BP.

Im kontinuierlichen Verfahren wird Titandioxid bevorzugt als Formkörper eingesetzt Die Verarbeitung von Titandioxid zu Formkörpern wird im folgenden als Konfektionierung bezeichnet. Titandioxid kann mittels unterschiedlicher Methoden konfektioniert werden. Die Konfektionierung von Titandioxid zu Katalysator-Formkörpern kann z.B. nach an sich bekannten Verfahren zur Herstellung von Katalysator-Formkörpern erfolgen, wie sie beispielsweise in Ertl, Knözinger, Weitkamp:"Handbook of Heterogenoeous Catalysis", VCH Weinheim, 1997, Seiten 98 ff beschrieben sind. Die Konfektionierung von Katalysator-Formkörpern aus Titandioxid ist beispielsweise auch aus US 4,388,288 bekannt. Gemäß den oben aufgeführten Schriften umfasst die Konfektionierung üblicherweise die Verfahrensschritte der Kompoundierung (Abmischung), der Formgebung sowie der Trocknung bzw. Kalzinierung.

Wie aus der aufgeführten Literaturstelle ebenfalls zu entnehmen ist, können bei der Kompoundierung Verarbeitungshilfsmittel, wie Bindemittel, Porenbildner und Anteigungsmittel sowie Wasser zum Titandioxid zugeben werden.

Der Anteil an Titandioxid in dem Katalysator-Formkörper kann in der Regel so gewählt werden, dass nach der im folgenden beschriebenen Trocknung und/oder Kalzinierung der Titangehalt im Katalysator-Formkörper mindestens 15 Gew.%, bevorzugt mindestens 30 Gew. % und besonders bevorzugt mindestens 50 Gew.%, jeweils bezogen auf die Gesamtmasse des Katalysator-Formkörpers beträgt. Der maximale Titangehalt in einem Katalysatorformkörper aus

reinem Titandioxid beträgt 59,95 Gew.%.

Durch den Prozess der Formgebung, der in den genannten Literaturstellen (Ertl, Knözinger, Weitkamp: "Handbook of heterogenoeous catalysis", VCH Weinheim, 1997, Seiten 98 ff, US 4,388,288) ebenfalls beschrieben ist, können Formkörper in jeglicher Raumform, z.B. rund, eckig, länglich oder dergleichen, z.B. in Form von Strängen, Tabletten, Granulat, Kugeln, Zylindern oder Körnern erhalten werden. Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.

Die untere Grenze der Formkörpergröße ist so gewählt, dass der durch den Strömungswiderstand hervorgerufene Druckaufbau aufgrund der erhöhten Festkörperdichte im Reaktor technisch kontrollierbar bleibt und die Formkörper noch eine ausreichende mechanische Stabilität aufweisen.

Die Größe der durch den Formgebungsprozess erhaltenen Titandioxid-Formkörper sollte nach dem nachfolgend beschriebenen Trocknungs- und/oder Kalzinierungsschritt vorzugsweise bei 0,5 bis maximal 10 mm, besonders bevorzugt bei 1 bis 5 mm liegen.

Nach dem Formgebungsprozess erfolgt in der Regel ein Trocknungs- und/oder Kalzinierungsschritt, bei denen der Formkörper üblicherweise bei höheren Temperaturen temperiert wird. Bei dem Trocknungs- und/oder Kalzinierungsschritt ist zu berücksichtigen, dass bei einer Reaktionstemperatur von mehr als 700°C die monotrope Umwandlung von Anatas zu Rutil erfolgt ("Industrial Inorganic Pigments", G. Buxbaum und G. Pfaff, Wiley-VCH, Weinheim, S. 51). Durch die Reaktionsdauer und die Reaktionstemperatur bei der Kalzinierung kann, falls gewünscht, das Verhältnis von Anatas zu Rutil eingestellt werden. In einer bevorzugten Ausführungsform wird der Trocknungs-und/oder Kalzinierungsschritt bei Temperaturen von unter 600°C durchgeführt, um eine Umwandlung von Anatas zu Rutil zu vermeiden. Besonders bevorzugt wird der Trocknungs- und/oder Kalzinierungsschritt bei Temperaturen von 200 bis 600°C durchgeführt.

Die Schneidhärte nach erfolgter Trocknung und/oder Kalzinierung von Formkörpern, die in Form von Strängen extrudiert wurden, kann im Bereich kleiner Werte, wie von 2 N bis 10 N, mittlerer Werte, wie größer 10 N bis 20 N, oder auch hoher Werte, wie größer 20 N oder größer 25 N, liegen. Die Bestimmung der Schneidhärte kann mittels der in dem Versuchsbeispiel beschriebenen Messmethode erfolgen.

Die BET-Oberfläche bestimmt gemäß der deutschen NORM DIN 66.131 nach der Mehrpunktsmethode mittels des volumetrischen Verfahrens, kann im Bereich von 10 bis 2000 $m^2/g$, vorzugsweise im Bereich 80 bis 120 $m^2/g$ liegen.

Ein Teil des Titandioxids kann durch andere Oxide und/oder Mischoxide ersetzt werden. Als solche kommen Oxide und/oder Mischoxid der Elemente Bor, Aluminium, Gallium, Indium, Silizium, Germanium, Zinn, Blei, Phosphor, Arsen, Antimon, Wismut, Scandium, Yttrium, Zirkon, Vanadium, Niob, Tantal oder Wolfram, bevorzugt Oxide und/oder Mischoxide der Elemente Bor, Aluminium, Gallium, Silizium, Zinn, Blei, Antimon, Wismut, Zirkon, Vanadium, Niob oder Wolfram, besonders bevorzugt kommen Oxide und/oder Mischoxide der Elemente Aluminium, Silizium, Zirkon, Vanadium oder Wolfram in Betracht. Der Anteil der obengenannten anderen Oxide und/oder Mischoxide kann in weiten Bereichen variieren. Er kann bis zu 80 Gew.% betragen, liegt aber bevorzugt bei 50 Gew.% oder darunter, besonders bevorzugt beträgt er nicht mehr als 20 Gew.%, jeweils bezogen auf die Gesamtmasse der eingesetzten Oxide bestehend aus Titandioxid und der obengenannten anderen Oxide und/oder Mischoxide.

Als Einsatzstoffe werden in dem erfindungsgemäßen Verfahren Benzophenone und Ammoniak eingesetzt. Benzophenone der allgemeinen Formel II können in der Schmelze oder in Lösung, bevorzugt in der Schmelze eingesetzt werden.

In der Verbindung sind m und n ganzzahlige Zahlen von 0 bis 5 und die Substituenten $R^1$ bzw. $R^2$ in der Verbindung I und II haben folgende Bedeutungen:

$R^1$ und $R^2$ können:

- $C_1$- bis $C_4$-Alkoxy wie Methoxy, Ethoxy, n-Propoxy, iso-Propoxy, n-Butoxy, iso-Butoxy, sec.-Butoxy oder tert.-Butoxy, bevorzugt Methoxy, Ethoxy, n-Propoxy" n-Butoxy, besonders bevorzugt Methoxy, Ethoxy,
- $C_1$- bis $C_2$-Alkylamin wie Methylamin oder Ethylamin, bevorzugt Methylamin,
- $C_1$- bis $C_4$-Dialkylamin wie Dimethylamin oder Diethylamin, bevorzugt Dimethylamin bedeuten.

$R^1$ und $R^2$ können unabhängig voneinander, gleich oder verschieden sein.

Sofern ein aromatischer Kern mit mehr als einem Substituenten $R^1$ bzw. mehr als einem Substituenten $R^2$ substituiert ist, können die Substituenten, die sich an einem Kern befinden entweder gleich oder verschieden voneinander sein.

Die Substituenten $R^1$ und $R^2$ können an jeder Position der aromatischen Kerne substituiert sein, bevorzugt werden die Substituenten $R^1$ und $R^2$ aber in der ortho- und/oder para-Stellung substituiert. Das Substitutionsmuster kann symmetrisch oder unsymmetrisch sein.

Besonders bevorzugt wird ein Benzophenon mit der CAS-Nummer 119-61-9 in die Reaktion eingesetzt, das

keine Substituenten hat und m und n einen Wert von Null aufweisen. Dieses Benzophenon wird bevorzugt in der Schmelze eingesetzt.

Als weiterer Einsatzstoff wird Ammoniak in der Reaktion eingesetzt. Ammoniak kann in flüssiger Form oder überkritisch eingesetzt werden. Bevorzugt wird Ammoniak in flüssiger Form eingesetzt.

In der Regel wird ein molarer Überschuss an Ammoniak zu Benzophenon der Formel II eingesetzt. Bevorzugt beträgt das molare Verhältnis von Ammoniak zu Benzophenon von 10 bis 100 zu 1, bevorzugt von 25 bis 50 zu 1 und besonders bevorzugt von 30 bis 40 zu 1.

Das Verfahren kann entweder diskontinuierlich, bevorzugt im Rührautoklaven, oder kontinuierlich, bevorzugt im Rohrreaktor, bevorzugt kontinuierlich im Rohrreaktor durchgeführt werden.

Die Temperaturen liegen im Allgemeinen in einem Bereich von 50 bis 150°C, bevorzugt von 80 bis 140°C, besonders bevorzugt bei 120 bis 140°C. Die Drücke liegen üblicherweise in einem Bereich von 50 bis 350 bar, bevorzugt von 150 bis 250 bar, besonders bevorzugt von 180 bis 220 bar.

Die im kontinuierlichem Betrieb eingestellte Katalysatorbelastung wird in der Regel so gewählt, dass eine Mindestkonzentration an Benzophenonimin. im Ausgangsstrom erhalten wird. In der Regel beträgt die Mindestkonzentration Benzophenonimin im Ausgangsstrom mehr als 80 Gew.%, bevorzugt mehr als 90 Gew.% und ganz besonders bevorzugt mehr als 95 Gew.%.

Die im folgenden angegebenen Bereiche für typische Katalysatorbelastungen sollten nicht als strenge Grenzen für die Belastbarkeit des Katalysators verstanden werden, sondern als Erfahrungswerte, die beispielsweise durch die Reaktionsbedingungen und die Reaktorgeometrie beeinflussbar sind. Die maximale Katalysatorbelastung kann aber vom Fachmann durch Versuche leicht festgestellt werden. Mindestkonzentrationen von 95 Gew.% Benzophenonimin können bei denen als besonders bevorzugt genannten Reaktionsbedingungen beispielsweise bei Belastungen von mindestens 0,1 kg BP pro Kilogramm Katalysator und Stunde (kg BP/($kg_{Kat}$*h)), beispielsweise 0,2 bis 3 kg BP pro Kilogramm Katalysator und Stunde (kg BP/($kg_{Kat}$*h)), bevorzugt 0,5 bis 2,5 kg BP/($kg_{Kat*}$h) und besonders bevorzugt zwischen 0,7 und 2,0 kg BP/($kg_{Kat}$*h) erhalten werden.

Im diskontinuierlichen Verfahren kann Titandioxid bevorzugt als Pulver eingesetzt werden. Das Verhältnis von Titandioxid-Pulver zu Benzophenon im diskontinuierlichen Verfahren sollte wie oben beschrieben in dem Bereich von 0,001 bis 10 kg Titandioxid/kg BP, bevorzugt in dem Bereich von 0,01 bis 3 kg Titandioxid/kg BP und besonders bevorzugt in dem Bereich von 0,5 bis 2 kg Titandioxid/kg BP liegen.

Es versteht sich von selbst, dass wenn die Aktivität des in die Reaktion eingesetzten Titandioxids nachlässt, eine Regenerierung, beispielsweise durch Waschen möglich ist. Für das Waschen können z.B. Flüssigkeiten, wie Ammoniak, Wasser oder Alkoholen, wie Methanol, Ethanol, oder Propanol verwendet werden. Geeignete Waschflüssigkeiten können auch Mischungen der vorstehend genannten Flüssigkeiten sein.

Benzophenonimine der Formel I können als Bausteine in der Synthesechemie verwendet werden. Insbesondere kann ein Benzophenonimin, das keine Substituenten hat und m und n Werte von Null annehmen, als Vorprodukt bei der Herstellung von Lichtschutzmitteln (z.B. 2-Cyano-3,3-diphenylacrylsäure-ethylester) [Bull. Chem. Soc. Fr. (1963) 1576-1583] verwendet werden.

Ein Vorteil des erfindungsgemäßen Verfahren ist, dass gegenüber dem Stand der Technik verbesserte Ausbeuten und Selektivitäten als bei herkömmlichen Verfahren erzielt werden.

Ein weiterer Vorteil des erfindungsgemäßen Verfahrens ist, dass bei einer kontinuierlichen Herstellung die Belastung des Katalysators mit Edukt gegenüber herkömmlichen Verfahren (Stand der Technik) ohne eine Verringerung des Umsatzes an Benzophenonen und ohne eine Verschlechterung der Selektivitäten erhöht werden kann. Dies ermöglicht beispielsweise eine Kapazitätserweiterung in bestehenden Anlagen bzw. eine Reduzierung des Reaktionsvolumens bei Neuanlagen und eine damit verbundene Reduktion der Investitionssumme unter Beibehaltung einer definierten Produktqualität. Unter Produktqualität wird im vorliegenden Fall der Mindestgehalt an Benzophenonimin und der Maximalgehalt an Benzophenon definiert, der nach Durchführung des erfindungsgemäßen Verfahrens im diskontinuierlichen Reaktor nach Beendigung der Reaktion bzw. am Ausgang des kontinuierlichen Reaktors erhalten wird. Die Anforderungen an die Produktqualität sind abhängig von der Weiterverwendung des Benzophenonimins und den damit verbundenen Kundenwünschen.

Bei den nachfolgend beschriebenen Versuchen wird im folgenden der Begriff Benzophenonimin für ein Benzophenonimin der Formel I verwendet, das keine Substituenten hat und m und n einen Wert von Null aufweisen. Weiterhin wird der Begriff Benzophenon für ein Benzophenon der Formel II verwendet, das keine Substituenten hat und m und n einen Wert von Null aufweisen (CAS-Nummer 119-61-9).

Als Maß für die Belastbarkeit der Katalysatoren wurde bei den nachfolgend beschriebenen, kontinuierlich betriebenen Versuchen im Rohrreaktor eine Mindestkonzentration von Benzophenonimin von 95% im Ausgangsstrom als Spezifikationsgrenze zugrunde gelegt. Die Belastung des Katalysators mit Benzophenon wurde in den kontinuierlichen Versuchen so lange gesteigert, bis die Mindestkonzentration von 95% an Benzophenonimin unterschritten wurde. Die Belastung, bei der gerade noch die Spezifikation bezüglich der Mindestkonzentration Benzophenonimin (95%) im Ausgangsstrom erreicht wurde, wird in den folgenden Versuchen als maximale Belastbarkeit definiert. Die Gehalte an

Benzophenon und Benzophenonimin sowie von Nebenprodukten wurden mittels Gaschromatographie als Flächenprozente (F%) ermittelt. Hierbei beziehen sich die Flächenprozente der Signale auf die Gesamtfläche unterhalb der gemessenen Signale mit Ausnahme des Wassersignals.

Der Benzophenon-Umsatz U(BP) berechnet sich nach folgender Formel:

$$U(BP) = \frac{F\%(BP)_{Anfang} - F\%(BP)_{Ende}}{F\%(BP)_{Anfang}}$$

Die Ausbeute an Benzophenonimin A(BPI) ergibt sich aus den Flächenprozenten des Benzophenon-Signals.

$$A(BPI) = F\%(BPI)$$

Die Selektivität von Benzophenonimin S(BPI) berechnet sich als Quotient von Benzophenonimin-Ausbeute und Benzophenon-Umsatz:

$$S(BPI) = \frac{A(BPI)}{U(BP)}$$

Die Erfindung wird in den nachfolgenden Beispielen erläutert.

Messung der Schneidhärte:

Die Messung der Schneidhärte erfolgte mit einem Gerät der Fa. Zwick-Roell vom Typ BZ 2.5/TS1S. Die Vorkraft betrug 0,5 N, die Vorkraft-Geschwindigkeit 10 mm/min und die Prüfgeschwindigkeit 1,6 mm/min. Die Breite der Klinge des verwendeten Messers betrug 0,6 mm. Der angegebene Messwert ist der Mittelwert aus einer Prüfung von 30 Katalysatorformkörpern, die eine Strangform aufwiesen.

Beispiel 1 (Erfindungsgemäßes Verfahren - Kontinuierlich):

Ein Rohreaktor wurde mit 500 ml Katalysator-Formkörper (Extrudaten) mit einem Durchmesser von 1,5 mm befüllt. Als Maß für den Anteil an Titandioxid im Katalysator-formkörper wurde der Titangehalt bestimmt. Der Katalysatorformkörper bestand aus reinem Titandioxid. Das Titandioxid lag zu einem Anteil von mehr als 99% in der Anatas-Modifikation vor. Die Reaktion wurde bei einer Temperatur von 120°C und einem Druck von 200 bar durchgeführt. Der Reaktor wurde mit verschiedenen Belastungen zwischen 0,3 kg BP/($kg_{Kat}$*h) und 1,2 kg BP/($kg_{Kat}$*h) gefahren (siehe Tabelle 1). Das molare Einsatzstoffverhältnis von Ammoniak zu Benzophenon betrug 38:1. Die BET-Oberfläche gemessen nach DIN 66.131 betrug 100 $m^2$/g. Das Porenvolumen gemessen mittels Quecksilber-Porosimetrie gemäß DIN 66133 betrug 0,33 mL/g. Die Schneidhärte betrug 10 NM.

Tabelle 1:

| Belastung [kg BP/ ($kg_{Kat}$*h)] | Benzophenon-Umsatz [%] | Benzophenonimin-Selektivität [%] | Benzophenonimin-Ausbeute [%] |
|---|---|---|---|
| 0,5 | 94,9 | 99,8 | 94,7 |
| 0,6 | 95,1 | 99,9 | 95,0 |
| 0,7 | 95,0 | 99,9 | 95,0 |
| 0,8 | 95,1 | 99,9 | 95,0 |
| 1,0 | 95,1 | 99,9 | 95,0 |

(fortgesetzt)

| Belastung [kg BP/ ($kg_{Kat}$*h)] | Benzophenon-Umsatz [%] | Benzophenonimin-Selektivität [%] | Benzophenonimin-Ausbeute [%] |
|---|---|---|---|
| 1,2 | 95,1 | 99,9 | 95,0 |

Beispiel 1 zeigt, dass selbst bei einer Belastung von 1,2 kg BP/($kg_{Kat*}$h) die maximale Belastung noch nicht erreicht wurde. In den Versuchen war eine höhere Belastung des Katalysators durch die eine bestehende Limitierung hinsichtlich der Förderrate von Benzophenon nicht möglich. Mit den erfindungsgemäßen Katalysatoren können somit Belastungen von deutlich mehr als 0,6 kg BP/($kg_{Kat}$*h) erreicht werden und wenn keine apparatetechnischen Limitierungen bestehen, sogar mit mehr als 1,2 kg BP/($kg_{Kat}$*h) erreicht werden. Vergleichsweise, nahm in EP-A-0 713 861 der Benzophenon-Umsatz mit steigender Belastung bereits bei einer Katalysatorbelastung von 0,6 kg BP/($kg_{Kat}$*h) ab.

## Patentansprüche

1. Verfahren zur Herstellung von Benzophenonimin (BPI) der allgemeinen Formel I

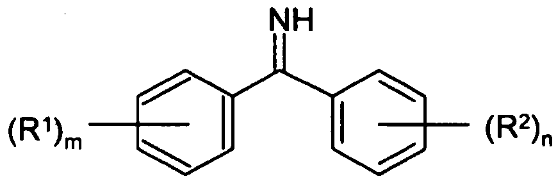

in der $R^1$ und $R^2$ $C_1$- bis $C_4$-Alkoxy, $C_1$- bis $C_2$-Alkylamin und $C_2$- bis $C_4$-Dialkylamin und m und n ganze Zahlen von 0 bis 5 bedeuten und $R^1$ und $R^2$ unabhängig voneinander gleich oder verschieden sein können, in dem man Benzophenon (BP) der allgemeinen Formel II

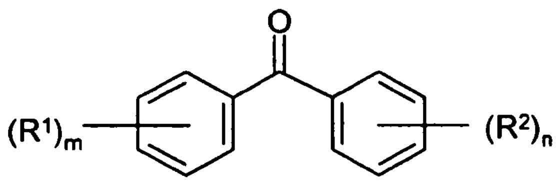

in der $R^1$, $R^2$, m und n die oben genannten Bedeutungen haben, in Ammoniak und in Gegenwart von Titandioxid umsetzt, wobei das Titandioxid zu mindestens 50 Gew.-% in der Anatas-Modifikation vorliegt.

2. Verfahren nach Anspruch 1, **dadurch gekennzeichnet, dass** man ein Benzophenon der Formel II einsetzt, das keine Substituenten hat und m und n einen Wert von Null aufweisen.

3. Verfahren nach mindestens einem der Ansprüche 1 bis 2, **dadurch gekennzeichnet, dass** ein Teil des Titandioxids durch mindestens ein Oxid und/oder Mischoxid aus der Gruppe bestehend aus den Elementen Bor, Aluminium, Gallium, Indium, Silizium, Germanium, Zinn, Blei, Phosphor, Arsen, Antimon, Wismut, Scandium, Yttrium, Zirkon, Vanadium, Niob, Tantal oder Wolfram ersetzt wird.

4. Verfahren nach mindestens einem der Ansprüche 1 bis 3, **dadurch gekennzeichnet, dass** das Titandioxid als Formkörper mit einer Größe von 0,5 mm bis 10 mm eingesetzt wird.

5. Verfahren nach mindestens einem der Ansprüche 1 bis 4, **dadurch gekennzeichnet, dass** die Herstellung der Benzophenonimine kontinuierlich erfolgt.

6. Verfahren nach mindestens einem der Ansprüche 1 bis 3, **dadurch** gekennzeichnent, dass das Titandioxid als

Pulver in die Reaktion eingesetzt wird.

7. Verfahren nach Anspruch 6. **dadurch gekennzeichnet, dass** das Verfahren diskontinuierlich in einem Rührautoklaven durchgeführt wird.

8. Verfahren zur Herstellung von Lichtschutzmitteln aus Benzophenonimin (BPI), **dadurch gekennzeichnet, dass** die Herstellung des Benzophenonimins durch ein Verfahren nach mindestens einem der Ansprüche 2 bis 7 erfolgt.

9. Verfahren nach Anspruch 8, **dadurch gekennzeichnet, dass** das Lichtschutzmittel 2-Cyano-3,3-diphenylacrylsäure-ethylester ist.

## Claims

1. A process for the preparation of benzophenonimine (BPI) of the general formula I

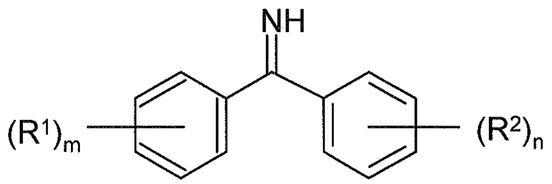

where $R^1$ and $R^2$ are $C_1$- to $C_4$-alkoxy, $C_1$- to $C_2$-alkylamine and $C_2$- to $C_4$-dialkylamine and m and n are integers from 0 to 5 and $R^1$ and $R^2$, independently of one another, may be identical or different, by reacting benzophenone (BP) of the general formula II

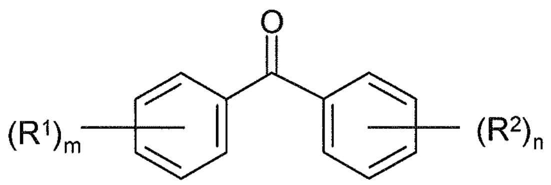

where $R^1$, $R^2$, m and n have the abovementioned meanings, in ammonia and in the presence of titanium dioxide, the titanium dioxide being present to an extent of at least 50% by weight in the anatase modification.

2. The process according to claim 1, wherein a benzophenone of the formula II which has no substituents and in which m and n have a value of zero is used.

3. The process according to at least one of claims 1 and 2, wherein a part of the titanium dioxide is replaced by at least one oxide and/or mixed oxide from the group consisting of the elements boron, aluminum, gallium, indium, silicon, germanium, tin, lead, phosphorus, arsenic, antimony, bismuth, scandium, yttrium, zirconium, vanadium, niobium, tantalum or tungsten.

4. The process according to at least one of claims 1 to 3, wherein the titanium dioxide is used in the form of moldings having a size of from 0.5 mm to 10 mm.

5. The process according to at least one of claims 1 to 4, wherein the preparation of the benzophenonimines is effected continuously.

6. The process according to at least one of claims 1 to 3, wherein the titanium dioxide is used as powder in the reaction.

7. The process according to claim 6, wherein the process is carried out batchwise in a stirred autoclave.

8. A process for the preparation of light stabilizers from benzophenonimine (BPI), wherein the preparation of the benzophenonimine is carried out by a process according to at least one of claims 2 to 7.

9. The process according to claim 8, wherein the light stabilizer is ethyl 2-cyano-3,3-diphenylacrylate.

**Revendications**

1. Procédé pour la préparation de benzophénone-imines (BPI) de formule générale I

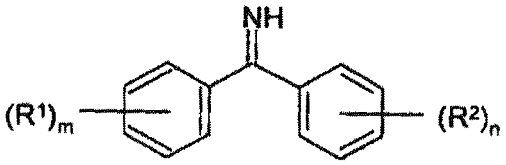

dans laquelle $R^1$ et $R^2$ représentent un groupe alcoxy en $C_1$-$C_4$, alkyl ($C_1$-$C_2$) amino ou dialkyl($C_2$-$C_4$)amino et m et n représentent des nombres entiers de 0 à 5 et $R^1$ et $R^2$ peuvent être identiques ou différents l'un de l'autre, dans lequel on fait réagir une benzophénone (BP) de formule générale II

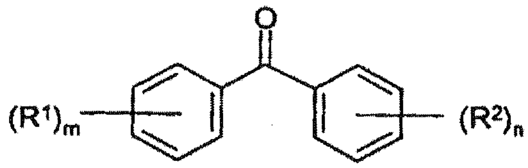

dans laquelle $R^1$, $R^2$, m et n ont les significations données ci-dessus, dans de l'ammoniac et en présence de dioxyde de titane, le dioxyde de titane étant présent à raison d'au moins 50 % en poids sous la forme anatase.

2. Procédé selon la revendication 1, **caractérisé en ce qu'**on utilise une benzophénone de formule II qui ne porte pas de substituants et m en n ont une valeur de zéro.

3. Procédé selon au moins l'une des revendications 1 et 2, **caractérisé en ce qu'**une partie du dioxyde de titane est remplacée par au moins un oxyde et/ou oxyde mixte choisi dans le groupe constitué par les éléments bore, aluminium, gallium, indium, silicium, germanium, étain, plomb, phosphore, arsenic, antimoine, bismuth, scandium, yttrium, zirconium, vanadium, niobium, tantale et tungstène.

4. Procédé selon au moins l'une des revendications 1 à 3, **caractérisé en ce que** le dioxyde de titane est utilisé sous forme de corps moulés ayant une taille de 0,5 mm à 10 mm.

5. Procédé selon au moins l'une des revendications 1 à 4, **caractérisé en ce que** la préparation des benzophénone-imines s'effectue en continu.

6. Procédé selon au moins l'une des revendications 1 à 3, **caractérisé en ce que** le dioxyde de titane est utilisé sous forme de poudre dans la réaction.

7. Procédé selon la revendication 6, **caractérisé en ce que** le procédé est effectué en mode discontinu dans un autoclave à agitation.

8. Procédé pour la préparation de photoprotecteurs à base de benzophénone-imine (BPI), **caractérisé en ce qu'**on effectue la préparation de la benzophénone-imine par un procédé selon au moins l'une des revendications 2 à 7.

9. Procédé selon la revendication 8, **caractérisé en ce que** le photoprotecteur est le 2-cyano-3,3-diphénylacrylate d'éthyle.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0688764 A1
- EP 0713861 A
- US 4388288 A


### In der Beschreibung aufgeführte Nicht-Patentliteratur


- Pigments, inorganic. Ullmann's Encyclopedia of Industrial Chemistry. 2000
- Titanium Dioxide. *CEH Marketing Research Report,* 2005
- Industrial Inorganic Pigments. Wiley VCH, 2005
- **Ertl ; Knözinger ; Weitkamp.** Handbook of heterogenoeous catalysis. VCH, 1997, 98 ff
- **G. Buxbaum ; G. Pfaff.** Industrial Inorganic Pigments. Wiley-VCH, 51